# EUROPEAN PATENT APPLICATION

(11) **EP 4 726 736 A1**
(43) Date of publication of application: **15.04.2026**
(21) Application number: 24205602.6
(22) Date of filing: 09.10.2024
(51) Int. Cl.: G16H 40/60

(54) **CONTROLLING POWER CONSUMPTION WITHIN A MEDICAL IMAGING ENVIRONMENT**

(71) Applicant: Koninklijke Philips N.V., 5656 AG Eindhoven (NL)
(72) Inventor: HENDRIKS, Bernardus Hendrikus Wilhelmus, Eindhoven (NL); NETO FONSECA, Lucia Teresa, Eindhoven (NL); SCHALK, Stefan Gerard, 5656AG Eindhoven (NL); BOON, Sjirk Niels, Eindhoven (NL); CULLA, Jaddy Glayd, Eindhoven (NL); ELENBAAS, Thijs, Eindhoven (NL)
(74) Representative: Philips Intellectual Property & Standards

(57) **Abstract**

A computer-implemented method of controlling power consumption within a medical imaging environment (110), is provided. The method includes: receiving video data (120) capturing at least a portion of a medical imaging system (130₁) within the medical imaging environment (110). The method also includes predicting, based on the video data (120), and for at least one electrical system (130_{1..i}) within the medical imaging environment including the medical imaging system (130₁), or a subsystem of the medical imaging system (130₁), a future demand for the at least one electrical system. The method also includes generating control signals (140_{1..i}) for controlling an energy consumption of the at least one electrical system (130_{1..i}) in accordance with the corresponding predicted future demand.

## Description

### TECHNICAL FIELD

The present disclosure relates to controlling power consumption within a medical imaging environment. A computer-implemented method, a computer program product, and a system, are disclosed.

### BACKGROUND

The power requirements of electrical systems that are used in medical imaging environments can be significant. For instance, the combined power requirement of an X-ray imaging system and its associated patient bed, medical image processing, monitors, interventional devices, and so forth, that are used in an interventional procedure can be in the order of 3.5kW. However, maintaining all of these electrical systems in a state of full operational readiness can be inefficient in energy terms because there are periods during which at least some of the electrical systems are not used.

In order to improve their energy efficiency, many electrical systems can be put into a so-called energy-saving mode, such as a standby mode, or they can be completely switched-off. However, it can also be challenging to determine when to implement such measures in medical imaging environments. For instance, a straightforward policy of putting an electrical system into an energy-saving mode, or switching it off, at night or during the weekend, can result in sub-optimal energy savings because there may be further opportunities to reduce energy consumption at other times. A policy of putting an electrical system into an energy-saving mode, or switching it off, immediately after use can also be problematic because there is typically a delay between the switching of an electrical system into an active state and the system reaching a state of full operational readiness. Such delays can interrupt the workflow in a medical imaging environment. A further complication of the latter policy is that some electrical systems consume a higher amount of energy during a startup period than they would otherwise consume during an equivalent period of full operational readiness. Thus, switching an electrical systems into a standby mode, or switching it off, for short periods, can be counterproductive in terms of saving energy.

Consequently, there remains a need to improve the control of power consumption of electrical systems within medical imaging environments.

### SUMMARY

According to one aspect of the present disclosure, a computer-implemented method of controlling power consumption within a medical imaging environment, is provided. The method includes:
receiving video data capturing at least a portion of a medical imaging system within the medical imaging environment;
predicting, based on the video data, and for at least one electrical system within the medical imaging environment including the medical imaging system, or a sub-system of the medical imaging system, a future demand for the at least one electrical system; and
generating control signals for controlling an energy consumption of the at least one electrical system in accordance with the corresponding predicted future demand.

In the above method, a future demand is predicted for electrical system(s) within a medical imaging environment. The future demand is predicted for at least a medical imaging system, or a sub-system of the medical imaging system, within the medical imaging environment. Control signals for controlling an energy consumption of the electrical system(s) are generated in accordance with the predicted future demand. Since the future demand is predicted based on video data that captures at least a portion the medical imaging system, the predictions are provided in a way that reflects the current usage of the medical imaging system. The control signals for the electrical system(s) in the medical imaging environment are therefore provided in a reliable manner. This in-turn has the dual effect of improving energy efficiency in the medical imaging environment, and also reducing workflow interruptions that might otherwise arise from delays incurred between the switching of the electrical system(s) into an active state and the system(s) reaching a state of full operational readiness.

Further aspects, features, and advantages of the present disclosure will become apparent from the following description of examples, which is made with reference to the accompanying drawings.

### BRIEF DESCRIPTION OF THE DRAWINGS

Fig. 1 is a flowchart illustrating an example of a computer-implemented method of controlling power consumption within a medical imaging environment, in accordance with some aspects of the present disclosure.
Fig. 2 is a schematic diagram illustrating an example of a system 200 for controlling power consumption within a medical imaging environment, in accordance with some aspects of the present disclosure.
Fig. 3 is a schematic diagram illustrating examples of various control signals 140_{1..i} for controlling an energy consumption of electrical systems within a medical imaging environment, in accordance with some aspects of the present disclosure.
Fig. 4 is a schematic diagram illustrating examples of various control signals 140₁', 140₁", 140₁" for controlling an energy consumption of sub-systems of an X-ray imaging system, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer-implemented method, may be implemented in a computer program product, and in a system, in a corresponding manner.

In the following description, reference is made to examples of a computer-implemented method of controlling power consumption within a medical imaging environment. The medical imaging environment includes at least one electrical system, including a medical imaging system. Reference is made to examples in which the medical imaging environment is an operating room and the medical imaging system is an X-ray imaging system. However, it is to be appreciated that in general, the computer-implemented method may be used to control the power consumption of electrical systems within any environment in which a medical imaging system is located. Thus, instead of an operating room, the medical imaging environment may alternatively be another type of medical imaging environment such as an examination room, or a procedure room, and so forth. It is also to be appreciated that the computer-implemented method may be used to control power consumption within a medical imaging environment that includes a medical imaging system that is different to an X-ray imaging system. For instance, the medical imaging system may alternatively be a computed tomography "CT" imaging system, or a positron emission tomography "PET" imaging system, or a single photon emission computed tomography "SPECT" imaging system, or a magnetic resonance "MR" imaging system, or an ultrasound imaging system, or an intravascular ultrasound "IVUS" imaging system, or an Optical Coherence Tomography "OCT" imaging system, or an endoscopy imaging system, or a colonoscopy imaging system, or a bronchoscopy imaging system, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed in the computer-implemented methods disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations performed in the computer-implemented methods disclosed herein.

As mentioned above, there remains a need to improve the control of power consumption of electrical systems within medical imaging environments.

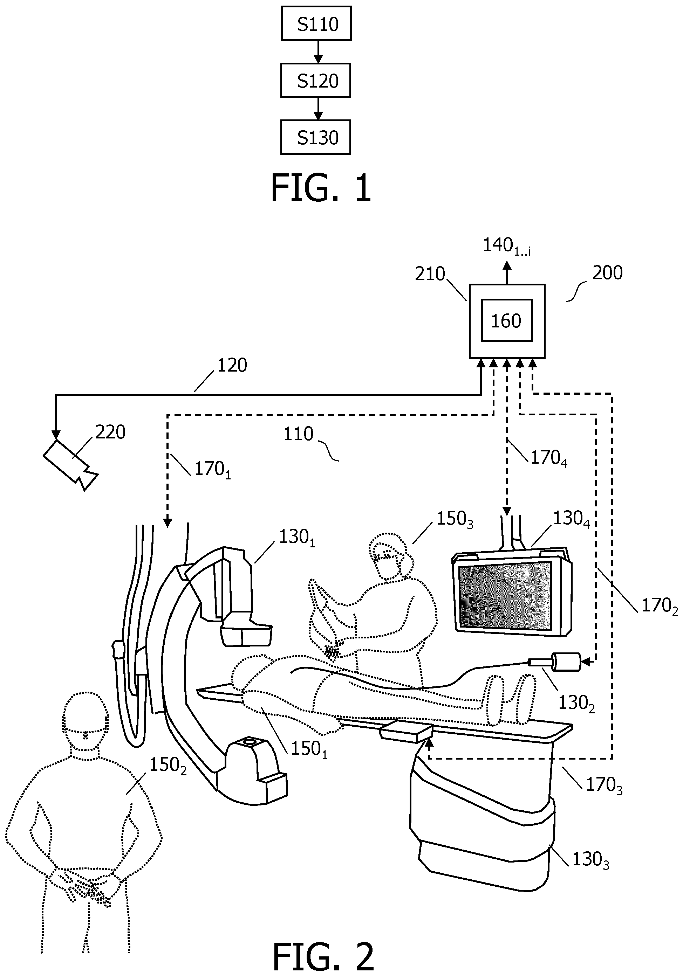

Fig. 1 is a flowchart illustrating an example of a computer-implemented method of controlling power consumption within a medical imaging environment, in accordance with some aspects of the present disclosure. Fig. 2 is a schematic diagram illustrating an example of a system 200 for controlling power consumption within a medical imaging environment, in accordance with some aspects of the present disclosure. It is noted that operations that are described in relation to the method illustrated in Fig. 1, may also be performed by the one or more processors 210 of the system 200 illustrated in Fig. 2. Likewise, operations that are described as being performed by the one or more processors 210 of the system 200 illustrated in Fig. 2, may also be performed in the method described with reference to Fig. 1. With reference to Fig. 1, and Fig. 2, the computer-implemented method of controlling power consumption within a medical imaging environment 110, includes:
receiving S110 video data 120 capturing at least a portion of a medical imaging system 130₁ within the medical imaging environment 110;
predicting S120, based on the video data 120, and for at least one electrical system 130_{1..i} within the medical imaging environment including the medical imaging system 130₁, or a sub-system of the medical imaging system 130₁, a future demand for the at least one electrical system; and
generating S130 control signals 140_{1..i} for controlling an energy consumption of the at least one electrical system 130_{1..i} in accordance with the corresponding predicted future demand.

In the above method, a future demand is predicted for electrical system(s) within a medical imaging environment. The future demand is predicted for at least a medical imaging system, or a sub-system of the medical imaging system, within the medical imaging environment. Control signals for controlling an energy consumption of the electrical system(s) are generated in accordance with the predicted future demand. Since the future demand is predicted based on video data that captures at least a portion the medical imaging system, the predictions are provided in a way that reflects the current usage of the medical imaging system. The control signals for the electrical system(s) in the medical imaging environment are therefore provided in a reliable manner. This in-turn has the dual effect of improving energy efficiency in the medical imaging environment, and also reducing workflow interruptions that might otherwise arise from delays incurred between the switching of the electrical system(s) into an active state and the system(s) reaching a state of full operational readiness.

The operations that are performed in the method illustrated in Fig. 1, are described in more detail below.

Referring initially to the operation S110 illustrated in Fig. 1; in this operation, video data is received. The video data captures at least a portion of a medical imaging system within a medical imaging environment.

In general, the medical imaging environment may be any environment in which a medical imaging system is located. For example, the medical imaging environment may be an examination room, or a procedure room, or an operating room. In general, the medical imaging system may be any type of medical imaging system. For example, the medical imaging system may be an X-ray imaging system, or a CT imaging system, or a PET imaging system, or a SPECT imaging system, or an MR imaging system, or an ultrasound imaging system, or an IVUS imaging system, or an OCT imaging system, or an endoscopy imaging system, or a colonoscopy imaging system, or a bronchoscopy imaging system.

An example of a medical imaging environment 110 is illustrated in Fig. 2. In this example, the medical imaging environment 110 is an operating room and the operating room includes a medical imaging system in the form of the X-ray imaging system 130₁.

With continued reference to the operation S110 illustrated in Fig. 1; the video data 120 that is received in this operation may be generated by various types of video cameras. For example, the video data 120 may be generated by a video camera that is sensitive to one or more optical wavelength intervals within the visible and/or infrared portion of the optical spectrum. The video data 120 may alternatively be generated by a different type of video camera, such as a hyperspectral camera, or a so-called depth camera. A depth camera generates video data that represents a range between the camera and objects within the camera's field of view. Various types of depth cameras are known, including depth cameras that employ a time-of-flight, or LIDAR principle, depth cameras that employ a structured light principle, and depth cameras that employ a binocular stereo vision principle. In the example illustrated in Fig. 2, video data 120 is generated by the video camera 220. In this example, the video camera 220 is sensitive to one or more optical wavelength intervals within the visible portion of the optical spectrum. The video data 120 captures at least a portion of the X-ray imaging system 130₁.

It is noted that the video data 120 that is received in the operation S110 may capture other information in addition to the at least a portion of the medical imaging system 130. For instance, in addition to the at least a portion of the medical imaging system 130, the video data 120 may additionally capture one or more persons within the medical imaging environment (e.g. one or more medical personnel interacting with the medical imaging system, one or more medical personnel interacting with a patient lying on a patient bed, a patient being imaged using the medical imaging system, a physician, and so forth). For example, with reference to Fig. 2, in addition to capturing at least a portion of the X-ray imaging system 130₁, the video data 120 that is captured by the video camera 220 may capture the clinician 150₃ performing a draping procedure on the patient 150₁ lying on the patient bed 130₃. Similarly, in addition to capturing at least a portion of the X-ray imaging system 130₁, the video data 120 that is captured by the video camera 220 may capture the physician 150₂.

With continued reference to the operation S110 illustrated in Fig. 1; the video data 120 that is received in this operation may be received by the one or more processors 210 that are illustrated in Fig. 2. In general, the video data 120 may be received by the one or more processors via any form of data communication. For instance, the video data may be received via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.

Referring now to the operation S120 illustrated in Fig. 1; in this operation, a future demand is predicted for at least one electrical system within the medical imaging environment including the medical imaging system 130₁, or a sub-system of the medical imaging system 130₁.

With reference to the example illustrated in Fig. 2, in the operation S120, a future demand may be predicted for the X-ray imaging system 130₁, or a sub-system of the X-ray imaging system 130₁, for example.

In some examples, the future demand is predicted for a plurality of electrical systems 130_{1..i} within the medical imaging environment 110 including the medical imaging system, or a sub-system of the medical imaging system. For instance, with reference to Fig. 2, the future demand may be predicted for the medical imaging system 130₁, or a subsystem thereof, and also for at least one of: a ventilation system (not illustrated in Fig. 2), a lighting system (not illustrated in Fig. 2), a heating system (not illustrated in Fig. 2), a patient bed 130₃, a monitor 130₄, a computing system (not illustrated in Fig. 2), a wireless communication system (not illustrated in Fig. 2), and an interventional device 130₂. Examples of interventional devices for which the future demand may be predicted include the contrast agent injector 130₂ illustrated in Fig. 2, and also other types of interventional devices such as a guidewire manipulator, a medical robot, a tissue sealing tool, and so forth.

Various examples of techniques for predicting of the future demand for an electrical system are described below. In these examples, the operation of predicting S120 a future demand for the at least one electrical system, may include predicting a future time at which the at least one electrical system will be required, or predicting a future time at which the at least one electrical system will not be required, or predicting a future time of an expected workflow phase to be performed using the medical imaging system, for example.

Referring now to the operation S130 illustrated in Fig. 1; in this operation, control signals 140_{1..i} are generated for controlling an energy consumption of the at least one electrical system 130_{1..i} in accordance with the corresponding predicted future demand.

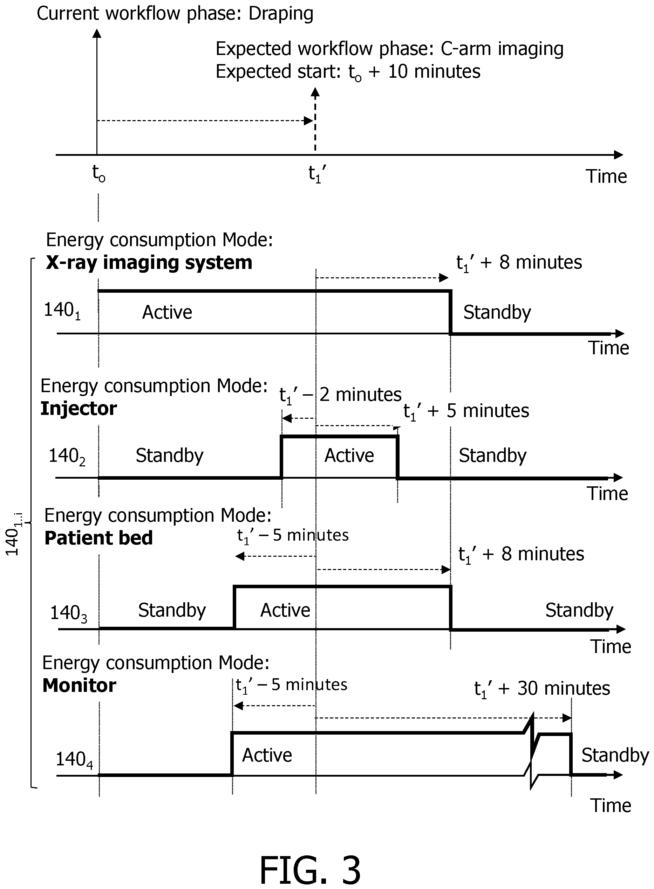

Fig. 3 is a schematic diagram illustrating examples of various control signals 140_{1..i} for controlling an energy consumption of electrical systems within a medical imaging environment, in accordance with some aspects of the present disclosure. Fig. 3 illustrates multiple control signals 140_{1..i} that may be used to control the corresponding electrical systems 130_{1..i} illustrated in Fig. 2. Thus, the control signal 140₁ may be used to control the X-ray imaging system 130₁ illustrated in Fig. 2, the control signal 140₂ may be used to control the injector 130₂ illustrated in Fig. 2, the control signal 140₃ may be used to control the patient bed 130₃ illustrated in Fig. 2, and the control signal 140₄ may be used to control the monitor 130₄ illustrated in Fig. 2.

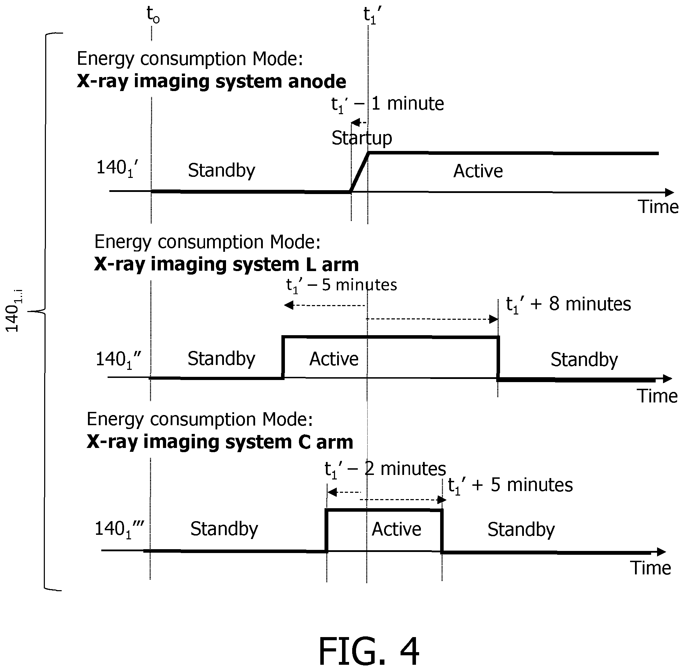

In general, the control signals that are generated in the operation S130 may be used to control the energy consumption of an entire electrical system, or they may be used to control the energy consumption of one or more sub-systems of an electrical system. For instance, the control signal 140₁ illustrated in Fig. 3 may be used to control the entire X-ray imaging system 130₁ illustrated in Fig. 2. Alternatively, the control signals that are generated in the operation S130 may be used to control the energy consumption of one or more sub-systems of the X-ray imaging system 130₁ illustrated in Fig. 2. This situation is described with reference to Fig. 4, which is a schematic diagram illustrating examples of various control signals 140₁', 140₁", 140₁" for controlling an energy consumption of sub-systems of an X-ray imaging system, in accordance with some aspects of the present disclosure. With reference to Fig. 4, the control signal 140₁' may be used to control a motor that drives a rotation of the anode of the X-ray tube of the X-ray imaging system illustrated in Fig. 2, the control signal 140₁" illustrated in Fig. 4 may be used to control the positioning of an X-ray imaging system L-arm that supports the C-arm of the X-ray imaging system illustrated in Fig. 2, and the control signal 140₁‴ illustrated in Fig. 4 may be used to control the positioning of the X-ray imaging system C-arm of the X-ray imaging system illustrated in Fig. 2. Other control signals (not illustrated in Fig. 4) may be used to control other sub-systems of the X-ray imaging system 130₁, such as a generator that provides a High Voltage to accelerate electrons between a cathode and an anode within the X-ray tube of the X-ray imaging system 130₁, or a processor that performs image reconstruction on image data that is acquired by the X-ray imaging system 130₁, and so forth.

In one example, the operation of generating S130 control signals 140_{1..i} for controlling an energy consumption of the at least one electrical system 130_{1..i} in accordance with the corresponding predicted future demand, includes generating control signals for controlling an energy consumption of the at least one electrical system in accordance with an energy consumption mode selected from a plurality of candidate energy consumption modes.

In this example, the use of various candidate energy consumption modes is contemplated. In general, there may be two candidate energy consumption modes, or there may be three or more candidate energy consumption modes. By way of some examples, the candidate energy consumption modes may include at least one of: a standby mode, a startup mode, an active mode, a shutdown mode, a relatively lower energy consumption mode, and a relatively higher energy consumption mode. Some examples of these energy consumption modes are illustrated in Fig. 3, which illustrates a Standby energy consumption mode, and an Active energy consumption mode; these being activated via the control signals 140_{1..i}. Fig. 4 illustrates an example of a Startup energy consumption mode that is activated via the control signal 140₁'.

By way of some further examples, the control signals may alternatively be used to control the X-ray imaging system anode of the X-ray imaging system 130₁ illustrated in Fig. 2, in accordance with the following three example candidate energy consumption modes. In a first "Shutdown" mode, the X-ray tube is shut down when no X-ray imaging is expected for at least the coming 60 seconds. In a second "Slow down" mode, the anode rotational speed is reduced to a level that facilitates relatively lower power imaging procedures such as fluoroscopic imaging but not relatively higher power imaging procedures, such as cine acquisition, digital subtraction angiography, single shot, 3D Rotational Angiography, and so forth. In a third "Full operational power" mode, the X-ray tube is ready to acquire images at full power at any time within the next 1 minute.

The control signals 140_{1..i} that are generated in the operation S130 may be outputted by the one or more processor(s) 210. The control signals may be outputted in various ways. For instance, in one example the control signals are outputted to the corresponding electrical system(s) in order to automatically control the energy consumption of the corresponding electrical system. In another example, the control signals are outputted to a display device, such as a monitor. In this latter example, the control signals, as indicated via the monitor, inform a user how to control the energy consumption of the corresponding electrical system(s).

As mentioned above, various techniques are contemplated for use in the operations S120 and S130 mentioned above, i.e. in predicting S120 a future demand for the at least one electrical system, and in generating S 130 control signals for controlling the energy consumption of the at least one electrical system in accordance with the corresponding predicted future demand. In general, these techniques include analyzing the video data 120 using various image processing techniques and/ or machine learning algorithms. In general, all that is required from the video data 120 in these techniques is that the video data captures at least a portion of the medical imaging system 130. This is because the video data, by capturing at least a portion of the medical imaging system, is representative of a usage of the medical imaging system. Even in examples in which predictions are made for the future demand of one or more electrical systems in addition to the medical imaging system, it is not essential that portions of these additional electrical systems are also captured in the video data 120. This is because predictions of the demand of these additional electrical systems can be made based on the predicted demand for the medical imaging system.

In one example, the video data 120 is analyzed in order to classify the activit(ies) of person(s) that are captured in the video data. Predictions of the future demand for the electrical system(s) are then made based on the classified activit(ies). In this example, the operation of predicting S120 a future demand for the at least one electrical system, comprises analyzing the video data 120 to classify an activity of one or more persons 150_{1..j} captured in the video data. The predicting S120 of the future demand, is then performed based on the classified activity.

In this example, the activities of person(s) captured in the video data may be classified into a set of categories such as "radiographer/ patient/ physician present in room", "patient lying on patient bed", "radiographer adjusting patient position", "draping", "C-arm imaging", "catheterization", and so forth.

The operation of classifying activities in this example may be performed using various image processing techniques. For example, the operation of analyzing the video data 120 may include inputting the video data into a machine learning algorithm 160 and classifying the activity of the one or more persons 150_{1..j} captured in the video data using the machine learning algorithm in response to the inputting. In this example, the machine learning algorithm 160 is trained to classify the activity of the one or more persons captured in the inputted video data 120.

The machine learning algorithm 160 in this example may be implemented by the one or more processors 210 illustrated in Fig. 2. The training data that is used to train the machine learning algorithm in this example may include training video data capturing usage of the medical imaging system. The training video data may capture instances of medical imaging procedures being performed using the medical imaging system, for example. The training video data may capture one or more persons within the medical imaging environment (e.g. one or more medical personnel interacting with the medical imaging system and/or medical personnel interacting with patient(s) lying on a patient bed and/or patient(s) being imaged using the medical imaging system). The training data may include ground truth activity data corresponding to the training video data, the ground truth activity data representing the corresponding activities. The training video data may be labelled with the ground truth activity data. For instance, ground truth activity labels may be applied to the training video data by a reviewer of the video data. The machine learning algorithm 160 in this example may be provided by a human activity detection model such as those described in a document by Nguyen, H. C., et al., "Deep learning for human activity recognition on 3D human skeleton: survey and comparative study", Sensors 23(2023)05121. Other examples of human activity detection models that may be used as the machine learning algorithm 160 in this example include the YOLO algorithm, AlphaPose, OpenPifPaf, and OpenPose.

In the above example, the operation of predicting S120 the future demand for the at least one electrical system based on the classified activities, may be performed in various ways. These include using the classified activit(ies) to consult a look-up table that provides an association between various activities and the expected future time(s) at which the various electrical system(s) will, or will not, be required. For instance, a look-up table may be used to predict that the onset of an X-ray imaging procedure is expected to occur after a specified delay of e.g. ten minutes, after the start of a draping procedure. The timings used in the look-up table may be known from empirical workflow data.

For instance, the activities of a person captured in the video data may be classified as "draping" wherein surgical drapes are applied to a patient in preparation for a surgical procedure. Having detected this activity, it may be predicted that a C-arm imaging procedure is likely to occur after a delay of say 10 minutes. This prediction may be made by using the classified activit(ies) to consult a look-up table that provides an association between various activities and the expected future time(s) at which the various electrical system(s) will, or will not, be required. By basing the predictions on classified activities, this example facilitates adjustments to the predictions to be made in a straightforward manner. For instance, if it becomes necessary to adjust the expected delay to 15 minutes, this adjustment can be made via the look-up table without having to adapt the classification of the activities.

Instead of using a look-up table to predict the future demand for the electrical system(s) in the operation S120, the machine learning algorithm 160 that classifies the activities may be trained to predict the future demand based on the classified activities. Thus, in another example, the machine learning algorithm 160 is further trained to predict for the classified activity, the corresponding future demand for the at least one electrical system 130_{1..i}. In this example, the operation of predicting S120 the future demand, is performed by the machine learning algorithm 160 in response to the inputting of the video data into the machine learning algorithm 160.

In this example, the training data that is used to train the machine learning algorithm 160 may include ground truth demand data corresponding to the ground truth activity data, the ground truth demand data representing, for each electrical system 130_{1..i}, a corresponding future demand for the electrical system. The training video data may be labelled with the ground truth demand data. For instance, the training video data may be labelled with ground truth labels that indicate a time period within which the medical imaging system and/or one or more further electrical systems are expected to be required.

In another example, rather than predicting the future demand from the classified activities in the operation S120, the future demand for the electrical system(s) is predicted directly from the video data 120. In this example, the operation of predicting S120 a future demand for the at least one electrical system, comprises inputting the video data 120 into a machine learning algorithm 160 and predicting the future demand using the machine learning algorithm in response to the inputting. In this example, the machine learning algorithm 160 is trained to predict the future demand from the inputted video data 120 using training video data and corresponding ground truth demand data, the training video data capturing usage of the medical imaging system 130₁, and the ground truth demand data representing for each electrical system 130_{1..i}, a corresponding future demand for the electrical system.

This example has the advantage that the future demand for the electrical system(s) is predicted directly from the video data 120. In this example, the training video data that is used to train the machine learning algorithm captures usage of the medical imaging system. The training video data may capture instances of medical imaging procedures being performed using the medical imaging system, for example. The training video data may capture one or more persons within the medical imaging environment (e.g. one or more medical personnel interacting with the medical imaging system and/or medical personnel interacting with patient(s) lying on a patient bed and/or patient(s) being imaged using the medical imaging system). The corresponding ground truth demand data that is used to train the machine learning model represents, for each electrical system 130_{1..i}, a corresponding future demand for the electrical system. For instance, the ground truth demand data may indicate a time period within which the medical imaging system and/or one or more further electrical systems are expected to be required. The training video data may be labelled with the ground truth demand data. For instance, the training video data may be labelled with ground truth labels that indicate a time period within which the medical imaging system and/or one or more further electrical systems are expected to be required. The machine learning model that is used in this example may be provided by a similar human activity detection models to those described above.

In a related example, the machine learning model is re-trained using further training video data that is acquired during a medical imaging procedure. In this example, the method includes re-training the machine learning algorithm 160 using further training video data and corresponding ground truth demand data acquired during a medical imaging procedure performed using the medical imaging system 130₁. The further training video data captures usage of the medical imaging system 130₁ during the medical imaging procedure, and the ground truth demand data represents, for each electrical system 130_{1..i}, a corresponding future demand for the electrical system.

In this example, the re-training of the machine learning algorithm helps to improve the accuracy of its predictions. In this example, the further training video data may capture instances of medical imaging procedures being performed using the medical imaging system, for example. The further training video data may capture one or more persons within the medical imaging environment (e.g. one or more medical personnel interacting with the medical imaging system and/or medical personnel interacting with patient(s) lying on a patient bed and/or patient(s) being imaged using the medical imaging system). The corresponding ground truth demand data that is used to re-train the machine learning model represents, for each electrical system 130_{1..i}, a corresponding future demand for the electrical system.

In this example, the future demand for an electrical system may be obtained from control data 170_{1..i} representing a manual switch-on, or a manual switch-off, of the corresponding electrical system 130_{1..i}. For instance, the one or more processors 210 illustrated in Fig. 2 may receive control data 170_{1..i} for the electrical systems 130_{1..i} illustrated in Fig. 2 and use this control data to re-train the machine learning algorithm 160.

In another example, the machine learning algorithms 160 described above are also configured to generate the control signals 140_{1..i} corresponding to the predicted future demand. Thus, the control signals may be generated directly by the machine learning algorithm 160. In this example, the machine learning algorithm is trained using training data that includes training video data and corresponding ground truth control signals.

In another example, the future demand for the electrical system(s) is predicted based on control data for the electrical system(s) as well as the video data 120. In this example, the method described with reference to Fig. 1 includes receiving control data 170_{1..i} representing a status of the at least one electrical system 130_{1..i}, and the operation of predicting S120 the future demand for the at least one electrical system, is performed based further on the control data 170_{1..i}.

Using the control data to predict the future demand in accordance with this example improves the reliability of the predictions that are made in the operation S120. In this example, the status may simply represent an "on versus off' status of the electrical system 130_{1..i}, or it may represent other status information such as an "in-use status versus a not in-use status" of the electrical system, or it may represent a current operation being performed by the electrical system. By way of some further examples, a status of the medical imaging system 130₁ may include an indication of a type of imaging procedure for which the medical imaging system is configured, an indication of a position of a C-arm of an X-ray imaging system, and so forth. Such status information may be obtained from log data of the medical imaging system. A status of a patient bed 130₃ may include an indication of a position of the patient bed.

In examples in which a machine learning algorithm 160 is used to predict the future demand, control data for the electrical system(s) that corresponds to the training video data may be inputted into the machine learning algorithm 160 during training. After training, control data that is acquired from the electrical system(s) is inputted into the machine learning algorithm and the machine learning algorithm bases its predictions in this control data.

In some examples, the operation of predicting S120 a future demand for at least one electrical system 130_{1..i} within the medical imaging environment, includes predicting a future time t₁' at which the at least one electrical system 130_{1..i} will be required and/or predicting a future time at which the at least one electrical system will not be required. In these examples, the corresponding operation of generating S130 control signals 140_{1..i} for controlling an energy consumption of the at least one electrical system 130_{1..i} in accordance with the corresponding predicted future demand, includes generating the control signals 140_{1..i} based on the respective predicted future time.

An example in which a future time is predicted at which an electrical system will be required, is now described with reference to Fig. 4. The current time is marked as to in the uppermost graph in Fig. 4. In the operation S120, and at time to, a future time t₁' is predicted at which the medical imaging system 130₁ will be required. This prediction may be made in accordance with the examples described above. For instance, the future time t₁' may be predicted based on the identification of a draping procedure in the video data 120. In the operation S130, the corresponding control signal 140₁' for controlling the rotation of the anode of the X-ray imaging system 130₁, is generated based on the predicted future time t₁'. Thus, at a time 1 minute prior to the time t₁', the control signal 140₁ switches the X-ray imaging system anode from the Standby mode to a Startup mode. During the Startup mode, the rotational speed of the anode of the X-ray imaging system is increased. A magnitude of a current applied to the cathode in the X-ray tube may also be increased. This prepares to X-ray imaging system for use. As a result, the X-ray imaging system is in the Active mode at the time t₁', which is the predicted time at which the at medical imaging system 130₁ will be required.

In another example, the operation of predicting S120 a future demand for the at least one electrical system 130_{1..i} within the medical imaging environment, comprises predicting a future time t₁' of an expected workflow phase to be performed using the medical imaging system 130₁. In this example, the operation of generating S130 control signals 140_{1..i} for controlling an energy consumption of the at least one electrical system 130_{1..i} in accordance with the corresponding predicted future demand, comprises generating the control signals based on the predicted future time t₁' of the expected workflow phase.

With reference to Fig. 3, in this example, in the operation S120, and at time to, a future time t₁' is predicted at which the workflow phase "C-arm imaging" is expected to occur. This prediction may be made using the example techniques described above. For instance, this prediction may be based on the detection of the current workflow phase as "Draping" in the video data, as illustrated in the upper graph in Fig. 3.

Fig. 3 illustrates examples of control signals that may be used to control multiple additional electrical systems in addition to the medical imaging system 130₁. As seen in Fig. 3, when the control signals 140_{1..i} are used to control energy consumption in multiple electrical systems, the control signals 140_{1..i} may control the energy consumption of the corresponding electrical systems such that the electrical systems are operated in different energy consumption modes at different times. Control signals for controlling one or more of such additional electrical systems may also be generated in accordance with the techniques described above. For instance, the control signal 140₂ illustrated in Fig. 3 may be generated and used to control the power consumption of the injector 130₂ illustrated in Fig. 2. As illustrated in Fig. 2, the control signal 140₂ may be used to switch the injector 130₂ into an Active state during the 7-minute period that extends from 2 minutes prior the expected time of the X-ray imaging system being required until a time 5 minutes after the X-ray imaging system is initially expected to be required. Similarly, the control signal 140₃ illustrated in Fig. 3 may be generated in accordance with the method and used to switch the patient bed 130₃ illustrated in Fig. 2 into an Active state during the 13-minute period that extends from 5 minutes prior to the expected time of the X-ray imaging system being required until a time 8 minutes after the X-ray imaging system is initially expected to be required. Similarly the control signal 140₄ illustrated in Fig. 3 may be generated in accordance with the method and used to switch the monitor 130₄ illustrated in Fig. 2 into an Active state during the 35-minute period that extends from 5 minutes prior the expected time of the X-ray imaging system being required, and until a time 30 minutes after the X-ray imaging system is initially expected to be required.

In general, the predictions for the future demand for these additional electrical systems, i.e. electrical systems in addition to the medical imaging system, are made based on the video data 120 that is received in the operation S110. These predictions can be made based on the predicted future demand for the medical imaging system, or they can be made as separate predictions, i.e. independently of the predicted future demand for the medical imaging system.

In another example, provision is made for over-riding of the control signals. In this example, the method illustrated in Fig. 1 includes:
receiving medical imaging system control data 170₁ representing a manual switch-on, or a manual switch-off, of the medical imaging system 130₁; and
over-riding the control signals 140₁ for the medical imaging system 130₁ such that the medical imaging system is operated in accordance with the respective manual switch-on, or the manual switch-off, of the medical imaging system.

The over-riding of the control signals provides a safety mechanism in the event of the need to immediately shut-down an electrical system, or alternatively the ability to immediately start-up an electrical system in an unexpected situation.

It is noted that in some situations, it may be desirable to maintain one or more of the electrical systems illustrated in Fig. 2 in an Active energy consumption mode at all times for safety reasons. For instance, instead of controlling the patient bed, and the L-arm, in accordance with the energy consumption modes described above, these electrical systems may be maintained in an Active energy consumption mode in-case it becomes necessary to immediately access a patient to provide a medical intervention.

In another example, a computer program product, is provided. The computer program product comprises instructions which when executed by one or more processors, cause the one or more processors to carry out a method of controlling power consumption within a medical imaging environment 110. The method includes:
receiving S110 video data 120 capturing at least a portion of a medical imaging system 130₁ within the medical imaging environment 110;
predicting S120, based on the video data 120, and for at least one electrical system 130_{1..i} within the medical imaging environment including the medical imaging system 130₁, or a sub-system of the medical imaging system 130₁, a future demand for the at least one electrical system; and
generating S130 control signals 140_{1..i} for controlling an energy consumption of the at least one electrical system 130_{1..i} in accordance with the corresponding predicted future demand.

In another example, a system 200 for controlling power consumption within a medical imaging environment 110, is provided. The system includes one or more processors 210 configured to:
receive S110 video data 120 capturing at least a portion of a medical imaging system 130₁ within the medical imaging environment 110;
predict S120, based on the video data 120, and for at least one electrical system 130_{1..i} within the medical imaging environment including the medical imaging system 130₁, or a sub-system of the medical imaging system 130₁, a future demand for the at least one electrical system; and
generate S130 control signals 140_{1..i} for controlling an energy consumption of the at least one electrical system 130_{1..i} in accordance with the corresponding predicted future demand.

An example of the system 200 is illustrated in Fig. 2. It is noted that in addition to the one or more processors 210, the system 200 may also include one or more of: a camera 220 for generating the video data; a display device such as a monitor 130₄ for displaying the control signals 140_{1..i}, other outputs generated by the one or more processors 120, and so forth; and a user input device (not illustrated in Fig. 2) configured to receive user input in relation to the operations performed by the one or more processors 210, such as a keyboard, a mouse, a touchscreen, and so forth.

The above examples are to be understood as illustrative of the present disclosure, and not restrictive. Further examples are also contemplated. For instance, the examples described in relation to a computer-implemented method, may also be provided by the computer program product, or by the computer-readable storage medium, or by the system 200, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of controlling power consumption within a medical imaging environment (110), the method comprising:
receiving (S110) video data (120) capturing at least a portion of a medical imaging system (130₁) within the medical imaging environment (110);
predicting (S120), based on the video data (120), and for at least one electrical system (130_{1..i}) within the medical imaging environment including the medical imaging system (130₁), or a sub-system of the medical imaging system (130₁), a future demand for the at least one electrical system; and
generating (S130) control signals (140_{1..i}) for controlling an energy consumption of the at least one electrical system (130_{1..i}) in accordance with the corresponding predicted future demand.

2. The computer-implemented method according to claim 1, wherein the predicting (S 120) comprises analyzing the video data (120) to classify an activity of one or more persons (150_{1..j}) captured in the video data; and
wherein the predicting (S120), is performed based on the classified activity.

3. The computer-implemented method according to claim 2, wherein the analyzing the video data (120) comprises inputting the video data into a machine learning algorithm (160) and classifying the activity of the one or more persons (150_{1..j}) captured in the video data using the machine learning algorithm in response to the inputting; and
wherein the machine learning algorithm (160) is trained to classify the activity of the one or more persons captured in the inputted video data (120).

4. The computer-implemented method according to claim 3, wherein the machine learning algorithm (160) is further trained to predict for the classified activity, the corresponding future demand for the at least one electrical system (130_{1..i}); and
wherein the predicting (S120) the future demand, is performed by the machine learning algorithm (160) in response to the inputting.

5. The computer-implemented method according to claim 1, wherein the predicting (S 120) comprises inputting the video data (120) into a machine learning algorithm (160) and predicting the future demand using the machine learning algorithm in response to the inputting; and
wherein the machine learning algorithm (160) is trained to predict the future demand from the inputted video data (120) using training video data and corresponding ground truth demand data, the training video data capturing usage of the medical imaging system (130₁), and the ground truth demand data representing for each electrical system (130_{1..i}), a corresponding future demand for the electrical system.

6. The computer-implemented method according to claim 5, wherein the method further comprises:
re-training the machine learning algorithm (160) using further training video data and corresponding ground truth demand data acquired during a medical imaging procedure performed using the medical imaging system (130₁), the further training video data capturing usage of the medical imaging system (130₁) during the medical imaging procedure, and the ground truth demand data representing for each electrical system (130_{1..i}), a corresponding future demand for the electrical system.

7. The computer-implemented method according to claim 4 or claim 5, wherein the machine learning algorithm (160) is further configured to generate the control signals (140_{1..i}) corresponding to the predicted future demand.

8. The computer-implemented method according to any previous claim, wherein the predicting (S120) comprises predicting a future time (t₁') at which the at least one electrical system (130_{1..i}) will be required and/or predicting a future time at which the at least one electrical system will not be required; and
wherein the generating (S130), comprises generating the control signals (140_{1..i}) based on the respective predicted future time.

9. The computer-implemented method according to any previous claim, wherein the predicting (S120) comprises predicting a future time (t₁') of an expected workflow phase to be performed using the medical imaging system (130₁); and
wherein the generating (S130) control signals (140_{1..i}) for controlling an energy consumption of the at least one electrical system (130_{1..i}) in accordance with the corresponding predicted future demand, comprises generating the control signals based on the predicted future time (t₁') of the expected workflow phase.

10. The computer-implemented method according to any previous claim, wherein the generating (S130) control signals (140_{1..i}) for controlling an energy consumption of the at least one electrical system (130_{1..i}) in accordance with the corresponding predicted future demand, comprises generating control signals for controlling an energy consumption of the at least one electrical system in accordance with an energy consumption mode selected from a plurality of candidate energy consumption modes.

11. The computer-implemented method according to claim 10, wherein the candidate energy consumption modes include at least one of: a standby mode, a startup mode, an active mode, a shutdown mode, a relatively lower energy consumption mode, and a relatively higher energy consumption mode.

12. The computer-implemented method according to any previous claim, wherein the predicting (S120) is performed for a plurality of electrical systems (130_{1..i}) within the medical imaging environment (110); and
wherein the plurality of electrical systems (130_{1..i}) further includes at least one of: a ventilation system, a lighting system, a heating system, a patient bed (130₃), a monitor (130₄), a computing system, a wireless communication system, and an interventional device (130₂).

13. The computer-implemented method according to any previous claim, wherein the method further comprises:
receiving medical imaging system control data (170₁) representing a manual switch-on, or a manual switch-off, of the medical imaging system (130₁); and
over-riding the control signals (140₁) for the medical imaging system (130₁) such that the medical imaging system is operated in accordance with the respective manual switch-on, or the manual switch-off, of the medical imaging system.

14. A computer program product comprising instructions which when executed by one or more processors, cause the one or more processors to carry out the method according to any one of claims 1 - 13.

15. A system (200) for controlling power consumption within a medical imaging environment (110), the system comprising one or more processors (210) configured to:
receive (S110) video data (120) capturing at least a portion of a medical imaging system (130₁) within the medical imaging environment (110);
predict (S120), based on the video data (120), and for at least one electrical system (130_{1..i}) within the medical imaging environment including the medical imaging system (130₁), or a sub-system of the medical imaging system (130₁), a future demand for the at least one electrical system; and
generate (S130) control signals (140_{1..i}) for controlling an energy consumption of the at least one electrical system (130_{1..i}) in accordance with the corresponding predicted future demand.
